# EUROPEAN PATENT SPECIFICATION

(11) **EP 1 919 517 B1**
(45) Date of publication and mention of the grant of the patent: **14.01.2009**
(21) Application number: 06764967.3
(22) Date of filing: 14.07.2006
(51) Int. Cl.: A61K 51/12

(54) **RADIOLABELLED NANOPARTICLES**
RADIOAKTIV MARKIERTE NANOTEILCHEN
NANO PARTICULES RADIO MARQUÉES

(30) Priority: 15.07.2005 GB 0514513
(43) Date of publication of application: 14.05.2008
(73) Proprietor: GE Healthcare Limited, Little Chalfont Buckinghamshire HP7 9NA (GB); University of Oxford, Oxford OX1 2JD (GB)
(72) Inventor: BEER, Paul, D., Oxford, Oxfordshire OX1 3QR (GB); LANKSHEAR, Michael, Oxford, Oxfordshire OX1 3QR (GB); DATTANI, Hema, Oxford, Oxfordshire OX1 4JD (GB); JACKSON, Alex, Amersham, Buckinghamshire HP7 9LL (GB); AVORY, Michelle, Amersham, Buckinghamshire HP7 9LL (GB)
(74) Representative: Canning, Lewis R.
(86) International application number: PCT/GB2006/002620
(87) International publication number: WO 2007/010211

(56) References cited:
- WO-A-20/05018681
- WO-A-20/05116226
- DE-A1- 19 933 346
- US-A1- 2002 015 679
- CANEPA MARINA ET AL: "The influence of core size on electronic coupling in shell-core nanoparticles: gold clusters capped with pyrenoxylalkylthiolate." PHOTOCHEMICAL & PHOTOBIOLOGICAL SCIENCES : OFFICIAL JOURNAL OF THE EUROPEAN PHOTOCHEMISTRY ASSOCIATION AND THE EUROPEAN SOCIETY FOR PHOTOBIOLOGY. NOV 2003, vol. 2, no. 11, November 2003 (2003-11), pages 1177-1180, XP002403818 ISSN: 1474-905X

## Description

### Field of the Invention.

The present invention relates to radiolabelled nanoparticles having a radioisotope non-covalently bonded thereto. The radiolabelled nanoparticles are useful as radiopharmaceuticals. Kits and methods of preparation of the radiolabelled nanoparticles are also disclosed.

### Background to the Invention.

Nanoparticles (NPs) are designated solid colloidal particles with a diameter ranging from 1 to 1000 nm. Literature precedent for radiolabelling of NPs describes ions or salts of radionuclides being trapped in the NP matrix during the formation of the NP, or via polymerisation of a radiolabelled monomer. These processes have the disadvantage that the radiolabel is present from the outset, hence all steps require radioactive handling techniques, expose the operator to radiation dose, and increase the volume of radioactive waste. A further problem is that during the preparation time for the radiolabelled NP, radioactive decay is occurring resulting in loss of imaging capability. Since NP preparation may take several hours, this is a problem for radionuclides with half-lives of the order of hours or minutes rather than days.

NPs have been labelled with ^{99m}Tc and ¹²⁵I but in each case the type ofNP required is very specific and the radionuclide incorporation is not very high [Ghanem et al, Int. J. Appl. Radiat. Isot., 44, 1219-1224 (1993) and Roland et al, J. Pharm. Sci., 78, 481-484 (1989)]. Nanoparticles may also be labelled by covalent binding to a bifunctional chelator [Ghanem *et al* above]. For steric reasons a linker is often used between the chelator and the NP, which adds to the overall synthetic difficulty and has limited suitability depending on the type of NP.

WO 02/32404 discloses nanoparticles having a core which is a semiconductor or metal linked to a plurality of carbohydrate ligands. The core can be doped with an NMR active material such as gadolinium or europium for *in vitro* or *in vivo* use. The carbohydrate can be isotopically labelled to facilitate detection of the nanoparticles.

WO 2005/018681 discloses nanoradiopharmaceuticals which are associated with an *in vivo* biological targeting ligand. The nanoparticles are prepared by the reduction of radionuclides in aqueous media, such that the radioisotope is present from the outset.

US 2005/0019257 A1 discloses radioactive copper magnetic nanoparticles which have a surfactant coating. Fatty acids are a preferred surfactant.

WO 2005/014051 discloses an oil-in-water emulsion which comprises lipid/surfactant-coated nanoparticles formed from an oil-like compound coupled to an atom with an atomic number (Z) above 36.

DE-199 33 346-A discloses a radiolabelled molecule covalently attached to a nanoparticle made of Polystyrol.

### The Present Invention.

Nanoparticles are used in nuclear medicine to deliver a radionuclide for imaging or therapy with the nature of the NP being used to adjust or target the biodistribution of the radionuclide *in vivo.* This may be achieved by tuning the variables defining the NP such as size, surface charge, matrix, surface coating, hydrophobicity and the inclusion of a targeting vector. Prior art methodology for tuning these physicochemical properties together with the need for covalent attachment of a biological targeting moiety and also radiolabelling together present a very difficult challenge.

The present invention provides radiolabelled NPs where the NP is synthesised non-radioactively as a first step. In this manner the properties of the NP (size, surface charge, and surface coating) may be varied without the complications associated with the radioisotope being present. As an optional second step, a biological targeting moiety may be introduced allowing the same NP to be used for different specific *in vivo* targeting applications. In the final step the NP is radiolabelled with high percentage incorporation, in a manner which permits a choice of radionuclides. Such a system offers unprecedented flexibility, simplifies the radiolabelling significantly and enables the use of non-radioactive kits for the preparation of NP radiopharmaceuticals.

### Detailed Description of the Invention.

In a first embodiment, the present invention provides a radiolabelled nanoparticle which comprises a nanoparticle having:
(i) a metallic core which comprises copper, silver, palladium or gold or combinations thereof;
(ii) a lipophilic coating around said core which comprises a multiplicity of C₂₋₂₅ organic thiols bound to said core, wherein said thiols may be the same or different and may be in reduced (ie. thiol) or oxidised (ie. disulfide) form or combinations thereof;
which is labelled with at least one radioisotope which is non-covalently bonded to said nanoparticle.

By the term "nanoparticle" is meant a particle which is approximately spherical in shape and in the size range 1 to 1000 nm.

By the term "metallic core" is meant a solid metal colloidal particle which forms the innermost part of the nanoparticle. Suitable core materials are the noble metals, in particular copper, silver, gold or palladium or combinations thereof. Preferred core materials are gold and silver, with the most preferred material being gold. Nanoparticles cores formed from mixtures of one or more of copper, silver, palladium and gold are also envisaged for use in the present invention. When such a mixture is employed, a preferred embodiment is the use of an alloy. Such alloys include: Au/Ag, Au/Cu and Au/Ag/Cu. The metallic core of the nanoparticles of the present invention is preferably non-radioactive.

The mean diameter of the core is preferably in the range 0.5 to 100 nm, more preferably in the range 1 to 50 nm, and most preferably in the range 1 to 20nm. The mean diameter can be measured using techniques well known in the art such as transmission electron microscopy.

By the term "organic thiol" is meant a compound having a thiol (-SH) group covalently bonded to a carbon atom of an alkyl or aryl or heteroaryl radical. The organic thiol of the present invention may be present in the reduced (ie. thiol) or oxidised (ie. disulphide) form, or combinations thereof. Preferably, the organic thiol is present in the reduced form. Where disulphides are used, it is anticipated that redox equilibria or *in situ* reduction generates the corresponding thiol or dithiol which is the preferred nanoparticle stabilising species.

By the term "non-covalently bonded" is meant bonding due to ion-pairing effects, hydrogen bonding, anion-pi aromatic or Lewis acid-base interactions in organic or aqueous media. This is to be contrasted with prior art approaches involving covalent bonding such as metal coordination compounds. The nature of the present nanoparticles is such that the radioisotope is outside the metallic core, and is either associated with the surface of the metal core, or the lipophilic coating.

By the term "biological targeting moiety" is meant: 3-100 mer peptides or peptide analogues which may be linear peptides or cyclic peptides or combinations thereof; enzyme substrates, antagonists or inhibitors; synthetic receptor-binding compounds; proteins or protein fragments; oligonucleotides, or oligo-DNA or oligo-RNA fragments.

By the term "cyclic peptide" is meant a sequence of 5 to 15 amino acids in which the two terminal amino acids are bonded together by a covalent bond which may be a peptide or disulphide bond or a synthetic non-peptide bond such as a thioether, phosphodiester, disiloxane or urethane bond. By the term "amino acid" is meant an *L*- or *D*-amino acid, amino acid analogue or amino acid mimetic which may be optically pure, i.e. a single enantiomer and hence chiral, or a mixture of enantiomers. Preferably the amino acids of the present invention are optically pure. By the term "amino acid mimetic" is meant synthetic analogues of naturally occurring amino acids which are isosteres, i.e. have been designed to mimic the steric and electronic structure of the natural compound. Such isosteres are well known to those skilled in the art and include but are not limited to depsipeptides, retro-inverso peptides, thioamides, cycloalkanes or 1,5-disubstituted tetrazoles [see M. Goodman, Biopolymers, 24, 137, (1985)].

Suitable peptides for use in the present invention include:
- somatostatin, octreotide and analogues,
- peptides which bind to the ST receptor, where ST refers to the heat-stable toxin produced by *E.coli* and other micro-organisms;
- laminin fragments eg. YIGSR, PDSGR, IKVAV, LRE and KCQAGTFALRGDPQG,
- N-formyl peptides for targeting sites of leucocyte accumulation,
- Platelet factor 4 (PF4) and fragments thereof,
- RGD (Arg-Gly-Asp)-containing peptides, which may eg. target angiogenesis [R. Pasqualini et al., Nat Biotechnol. 1997 Jun;15(6):542-6]; [E. Ruoslahti, Kidney Int. 1997 May;51(5):1413-7].
- peptide fragments of α₂-antiplasmin, fibronectin or beta-casein, fibrinogen or thrombospondin. The amino acid sequences of α₂-antiplasmin, fibronectin, beta-casein, fibrinogen and thrombospondin can be found in the following references: α₂-antiplasmin precursor [M.Tone et al., J.Biochem, 102, 1033, (1987)]; beta-casein [L.Hansson et al, Gene, 139, 193, (1994)]; fibronectin [A.Gutman et al, FEBS Lett., 207, 145; (1996)]; thrombospondin-1 precursor [V.Dixit et al, Proc. Natl. Acad. Sci., USA, 83, 5449, (1986)]; R.F.Doolittle, Ann. Rev. Biochem., 53, 195, (1984);
- peptides which are substrates or inhibitors of angiotensin, such as: angiotensin II Asp-Arg-Val-Tyr-Ile-His-Pro-Phe (E. C. Jorgensen et al, J. Med. Chem., 1979, Vol 22, 9, 1038-1044)
   [Sar, Ile] Angiotensin II: Sar-Arg-Val-Tyr-Ile-His-Pro-Ile (R.K. Turker et al., Science, 1972, 177, 1203).
- Angiotensin I: Asp-Arg-Val-Tyr-Ile-His-Phe-Phe-His-Leu.

Preferably the peptides of the present invention comprise antiplasmin or angiotensin II peptides. Antiplasmin peptides comprise an amino acid sequence taken from the N-terminus of
(i) α₂-antiplasmin,
   i.e. NH₂-Asn-Gln-Glu-Gln-Val-Ser-Pro-Leu-Thr-Leu-Thr-Leu-Leu-Lys-OH or variants of this in which one or more amino acids have been exchanged, added or removed such as:
   NH₂-Asn-GIn-Glu-GIn-Val-Ser-Pro-Leu-Thr-Leu-Thr-Leu-Leu-Lys-Gly-OH, NH₂-Asn-Gln-Glu-Ala-Val-Ser-Pro-Leu-Thr-Leu-Thr-Leu-Leu-Lys-Gly-OH, NH₂-Asn-Gln-Glu-Gln-Val-Gly-OH; or
(ii) casein
   ie. Ac-Leu-Gly-Pro-Gly-Gln-Ser-Lys-Val-Ile-Gly.

Synthetic peptides of the present invention may be obtained by conventional solid phase synthesis, as described in P. Lloyd-Williams, F. Albericio and E. Girald; Chemical Approaches to the Synthesis of Peptides and Proteins, CRC Press, 1997.

Suitable enzyme substrates, antagonists or inhibitors include glucose and glucose analogues such as fluorodeoxyglucose; fatty acids, or elastase, Angiotensin II or metalloproteinase inhibitors. A preferred non-peptide Angiotensin II antagonist is Losartan.

Suitable synthetic receptor-binding compounds include estradiol, estrogen, progestin, progesterone and other steroid hormones; ligands for the dopamine D-1 or D-2 receptor, or dopamine transporter such as tropanes; and ligands for the serotonin receptor.

The biological targeting moiety is preferably of molecular weight of less than 15,000, most preferably less than 10,000, ideally less than 5,000. Preferred biological targeting moieties are peptides, proteins or enzyme substrates, enzyme antagonists or enzyme inhibitors.

The organic thiol of the present invention preferably does not comprise a carbohydrate. By the term "carbohydrate" is meant a polysaccharide, oligosaccharide or monosaccharide.

The organic thiol of the present invention preferably comprises one or more anion-binding or cation-binding substituents. Suitable "anion-binding substituents" are either neutral or are positively-charged, and may act as hydrogen bond donor groups or electrostatically to bind the anion in a non-covalent manner. They include the following functional group substituents: amide, urea, thiourea, ammonium, guanidinium imidazolium, benzimidazolium, amidinium, thiouronium and pyrrole.

Certain elements, such as boron tin, silicon, mercury may also act as Lewis acidic anion receptors. In some circumstances, cations (eg. Na⁺) may be co-bound with the anion, so the phrase 'anion-binding substituent' does not preclude the presence of some cations together with the anions. The anion-binding substituent binds a radioisotope anion (eg. iodide or pertechnetate) non-covalently in such a way that the anion is stably-bound, and hence resistant to removal by eg. repeat washing with solvents or challenge with plasma proteins *in vivo* or *in vitro.*

Suitable "cation-binding substituents" are either neutral or negatively-charged, and may act as Lewis base donor groups or electrostatically to bind the cation in a non-covalent manner. They include the following functional group substituents: polyether (crown ether macrocycles, open chain analogues or combinations thereof); cryptands; calixarenes or quinones. The cation-binding substituent binds a radioisotope cation (eg. ²⁰¹T1 as T1⁺ or other radiometal ions) non-covalently in such a way that the cation is stably-bound, and hence resistant to removal by eg. repeat washing with solvents or challenge with plasma proteins *in vivo* or *in vitro*

Preferred positively-charged anion-binding substituents are of Formula -ER¹₃⁺ X⁻, where:
E is N or P;
R¹ is C₁₋₁₀ alkyl, which may be linear or branched; C₂₋₁₀ alkoxyalkyl; C₂₋₁₂ aryl or C₂₋₁₂ heteroaryl;
X is Hal, OH, PF₆, H₂PO₄, nitrate, C₁₋₈ carboxylate or C₁₋₈ sulfonate.
When X is C₁₋₈ carboxylate, preferred X groups are acetate or benzoate.

Especially preferred organic thiols are of Formula R²SH, when present in the reduced form or R²S-SR² when present in the disulphide form, wherein R² is C₅₋₂₄ alkyl, C₅₋₂₄ aralkyl, or C₅₋₁₂ aryl, and R² may optionally be substituted with one or more anion- or cation- binding substituents, as defined above. Preferred such anion- or cation-binding substituents are as defined above. Preferably, the organic thiol comprises an anion-binding substituent. Most preferably, the anion-binding substituent binds iodide, pertechnetate or perrhenate. As noted above, the organic thiol is preferably present in the reduced form, ie. is of formula R²SH.

Radioisotopes of the present invention are those suitable for radiopharmaceutical imaging of the mammalian body *in vivo* or suitable for radiopharmaceutical therapy of the mammalian body *in vivo.* Such isotopes are known in the art. Preferred radioisotopes are: ^{99m}Tc, ^{94m}Tc ¹⁸⁶Re, ¹⁸⁸Re, ¹²³I, ¹²⁴I, ¹²⁵I or ¹³¹I. The preferred chemical form of the radioisotope is:
(i) pertechnetate for technetium radioisotopes;
(ii) perrhenate for rhenium radioisotopes;
(iii) iodide ion for iodine radioisotopes.

This has the advantage that these are the chemical forms which are most readily available (eg. from ⁹⁹Mo/^{99m}Tc radioisotope generators). This means that the radioisotope can be used directly to radiolabel the nanoparticles, without any further chemical reaction. This simplification is an advantage over prior art methods, which may involve eg. the use of reducing agents or additional chemical processing in order to achieve radiolabelling.

Preferred nanoparticles of the present invention are doped with an organic cation chosen from quaternary ammonium salts, phosphonium salts, imidazolium, uronium, or other biocompatible organic cation. Preferably, the organic cation comprises C₄-C₁₆ alkyl chains, most preferably C₆-C₁₂ alkyl chains with C₈-C₁₀ alkyl chains being especially preferred. By the term "doped" is meant that the nanoparticles include a proportion of the organic cation in their make up suitably at a [organic cation] [organic thiol] molar ratio of about 1:5 to 1:20, preferably 1:8 to 1:12, most preferably 1:10. When the molar ratio is 1:10, this corresponds to a approximately one mole of organic cation per NP. When the organic cation is a quaternary ammonium salt, a preferred such salt is Aliquat 336 chloride.

The non-radioactive nanoparticles of the present invention can be obtained by the method of Brust et al [JCS, Chem. Commun., 801-802 (1994); *ibid* 1655-1656 (1995)]. Brust employs a biphasic system wherein a sodium borohydride reduction of AuCl₄⁻ is carried out in the presence of a stabilising ligand, normally an alkanethiol such as dodecanethiol. The nanoparticles produced by this method vary slightly in size, but all have a diameter below 10nm. Doped nanoparticles can be prepared by mixing the pre-formed nanoparticle with the required molar ratio of organic cation in a suitable solvent. Further details are given in Example 5.

Non-radioactive nanoparticles of the present invention further comprising a biological targeting moiety, can be prepared using substitution reactions, ie. displacement of a portion of the existing thiols of the NP lipophilic coating. This is described in the Experimental section. Some biological targeting moieties possess thiol functional groups (eg. comprise cysteine amino acids), and hence may not need to be functionalised further. In many cases, a thiol-derivatised biological targeting moiety will be necessary. Such thiol derivatisation can be achieved by reaction with 2-iminothiolane, as described by Mishra et al [Find.Exp.Clin.Pharmacol., 24(10) 653-660 (2002)] and McCall et al [Bioconj.Chem., 1(3) 222-226 (1990)], or by functionalisation with thioctic acid as described in the Examples. The thioctic acid method is preferred since it gives rise to a disulphide derivative, which forms a chelating dithiol at the metal surface, and hence is better able to displace monodentate thiols.

In a second embodiment, the present invention provides a radiopharmaceutical composition which comprises a plurality of the radiolabelled nanoparticles of the first embodiment, together with a biocompatible carrier, in a form suitable for mammalian administration. The "biocompatible carrier" is a fluid, especially a liquid, in which the radiolabelled nanoparticles can be suspended, such that the composition is physiologically tolerable, ie. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous solution of one or more tonicity-adjusting substances (eg. salts of plasma cations with biocompatible counterions), sugars (e.g. glucose or sucrose), sugar alcohols (eg. sorbitol or mannitol), glycols (eg. glycerol), or other nonionic polyol materials (eg. polyethyleneglycols, propylene glycols and the like). Preferably the biocompatible carrier is pyrogen-free water for injection or isotonic saline.

Such radiopharmaceuticals are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 cm³ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, or "unit dose" and are therefore preferably a disposable or other syringe suitable for clinical use. The pre-filled syringe may optionally be provided with a syringe shield to protect the operator from radioactive dose. Suitable such radiopharmaceutical syringe shields are known in the art and preferably comprise either lead or tungsten.
The radiopharmaceuticals of the present invention may be prepared from kits, as is described in the fifth embodiment below. Alternatively, the radiopharmaceuticals may be prepared under aseptic manufacture conditions to give the desired sterile product. The radiopharmaceuticals may also be prepared under non-sterile conditions, followed by terminal sterilisation using e.g. gamma-irradiation, autoclaving, dry heat or chemical treatment (e.g. with ethylene oxide). Preferably, the radiopharmaceuticals of the present invention are prepared from kits.

In a third embodiment, the present invention provides a method of preparation of the radiolabelled nanoparticle of the first embodiment, which comprises:
(i) provision of non-radioactive, unlabelled nanoparticles as described in the first embodiment;
(ii) optional purification of the nanoparticles from step (i);
(iii) reaction of the pre-formed nanoparticles from step (i) or step (ii) with a source of the radioisotope, such that the radioisotope is non-covalently bound to the nanoparticle.

When the radiolabelled nanoparticle comprises a biological targeting moiety, it is most conveniently introduced by first preparing unlabelled nanoparticles having a lipophilic coating comprising organic thiols. A thiol-derivatised or thiol-containing biological targeting moiety can then be used to displace a portion of the initial organic thiols, giving the desired product. In both situations, the radiolabelling step is the last one. One illustration of this, using thioctic acid conjugation, is shown in Scheme 1 for a biological targeting vector:

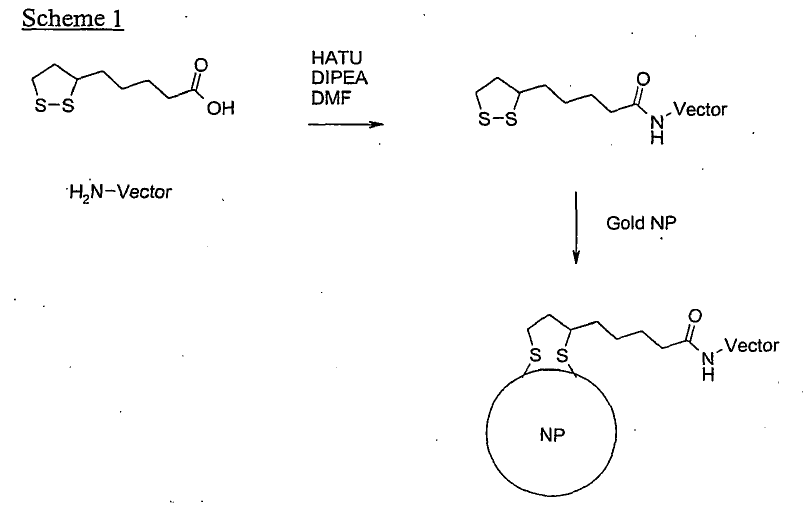

The present invention provides radiolabelled NPs where the NP is synthesised non-radioactively as a first step. In this manner the properties of the NP (size, surface charge, and surface coating) may be varied without the complications associated with the radioisotope being present. As an optional second step, a biological targeting moiety may be introduced allowing the same NP to be used for different specific *in vivo* targeting applications. In the final step the NP is radiolabelled with high percentage incorporation, in a manner which permits a choice of radionuclides. Such a system offers unprecedented flexibility, simplifies the radiolabelling significantly and enables the use of non-radioactive kits for the preparation of NP radiopharmaceuticals.

In the final step the NP is radiolabelled with high percentage incorporation, in a manner which permits a choice of radionuclides. An important feature is that the non-radioactive NP can be tailored to the most convenient chemical form of the radioisotope (eg. radioiodide or pertechnetate). This means that the radiolabelling can be carried out under very mild conditions, with minimal need for additional reagents such as reductants or oxidising agents. This maximises the convenience for the operator, and also minimises the risk of any inadvertent chemical degradation of the potentially-sensitive biological targeting moiety during the radiolabelling.

Once the radiolabelling has been carried out, purification of the radiolabelled NP, eg. to remove any unbound radioisotope, can be carried out as an optional additional step. Suitable purification methods are those which utilise the large difference in size between the nanoparticles and 'free' radioisotope to effect separation in the aqueous phase, without disrupting the nanoparticle. A preferred such method is Sephadex gel chromatography, using a Sephadex cartridge and is described in Example 9. ITLC also gave separation but is more suitable for analytical rather than preparative chromatography.

In a fourth embodiment, the present invention provides a kit for the preparation of the radiopharmaceutical composition of the second embodiment, which comprises the unlabelled nanoparticles of the first and third embodiments. Such kits comprise the unlabelled nanoparticles, preferably in sterile non-pyrogenic form, so that reaction with a sterile source of the radioisotope gives the desired radiopharmaceutical with the minimum number of manipulations. Such considerations are particularly important for radiopharmaceuticals where the radioisotope has a relatively short half-life, and for ease of handling and hence reduced radiation dose for the radiopharmacist. Hence, the reaction medium for reconstitution of such kits is preferably a "biocompatible carrier" as defined above, and is most preferably aqueous.

Suitable kit containers comprise a sealed container which permits maintenance of sterile integrity and/or radioactive safety, plus optionally an inert headspace gas (eg. nitrogen or argon), whilst permitting addition and withdrawal of solutions by syringe. A preferred such container is a septum-sealed vial, wherein the gas-tight closure is crimped on with an overseal (typically of aluminium). Such containers have the additional advantage that the closure can withstand vacuum if desired eg. to change the headspace gas or degas solutions.

The non-radioactive kits may optionally further comprise additional components such as a radioprotectant, antimicrobial preservative, pH-adjusting agent or filler.

By the term "radioprotectant" is meant a compound which inhibits degradation reactions, such as redox processes, by trapping highly-reactive free radicals, such as oxygen-containing free radicals arising from the radiolysis of water. The radioprotectants of the present invention are suitably chosen from: ascorbic acid, *para*-aminobenzoic acid (ie. 4-aminobenzoic acid), gentisic acid (ie. 2,5-dihydroxybenzoic acid) and salts thereof with a biocompatible cation. By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

By the term "antimicrobial preservative" is meant an agent which inhibits the growth of potentially harmful micro-organisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the radiopharmaceutical composition post-reconstitution, ie. in the radioactive diagnostic product itself. The antimicrobial preservative may, however, also optionally be used to inhibit the growth of potentially harmful micro-organisms in one or more components of the non-radioactive kit of the present invention prior to reconstitution. Suitable antimicrobial preservative(s) include: the parabens, ie. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [ie. *tris*(hydroxymethyl)aminomethane], and pharmaceutically acceptable bases such as sodium carbonate, sodium bicarbonate or mixtures thereof. When the conjugate is employed in acid salt form, the pH adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

The NPs for use in the kit may be employed under aseptic manufacture conditions to give the desired sterile, non-pyrogenic material. The NPs may also be employed under non-sterile conditions, followed by terminal sterilisation using e.g. gamma-irradiation, autoclaving, dry heat or chemical treatment (e.g. with ethylene oxide). Preferably, the NPs are employed in sterile, non-pyrogenic form. Most preferably the sterile, non-pyrogenic NPs are employed in the sealed container as described above.

In a fifth embodiment, the present invention provides the use of the radiolabelled nanoparticles of the first embodiment in the manufacture of a medicament for use in radiopharmaceutical imaging *in vivo.* Such radiopharmaceutical imaging is particularly useful for the diagnostic imaging *in vivo* of disease states of the mammalian body, wherein said mammal is previously administered with the radiopharmaceutical composition of the second embodiment. Since the NPs of the present invention have the flexibility to be adapted to a range of biological targets *in vivo*, a variety of imaging applications are possible.

By the term "previously administered" is meant that the step involving the clinician, wherein the imaging agent is given to the patient eg. intravenous injection, has already been carried out. This embodiment includes the use of the imaging agent of the first embodiment for the manufacture of diagnostic agent for the diagnostic imaging *in vivo* of disease states of the mammalian body.

In a sixth embodiment, the present invention provides the use of the radiolabelled nanoparticles of the first embodiment in the manufacture of a medicament for use in radiopharmaceutical therapy *in vivo.* Such radiopharmaceutical therapy is particularly useful in the treatment *in vivo* of disease states of the mammalian body, wherein said mammal is previously administered with the radiopharmaceutical composition of the second embodiment. The term "previously administered" is as defined above.

The invention is illustrated by the non-limiting Examples detailed below. Example 1 provides the preparation of gold nanoparticles with four different organic thiol coatings. Example 2 provides the syntheses of thiols with anion-binding amide substituents by reaction of thioctic acid with amines. Compound 2 was synthesised as a control, while Compounds 3 to 5 were designed to enhance the hydrophobicity of the anion binding site by providing either longer aliphatic chains or aromatic rings near the amide moiety. Compound 6 was designed to provide increased preorganisation when compared to the simpler monopodal ligands, and hence enhanced anion binding. Compounds 7 and 8 were designed to incorporate charge close to the amide moiety, which could enhance anion binding by electrostatic interactions, through protonation in the case of Compound 7 and by Na⁺ ion complexation in the case of Compounds 8.

The resultant amides both have two anion binding sites, but each also has another important feature. Compound 9 contains the adamantyl unit, which in addition to providing a hydrophobic environment in itself, has been previously shown to strongly bind within the cavity of β-cyclodextrins. Compound 10, was designed to permit examination of the anion binding event by fluorimetry, as the anthracene moiety is a well known fluorophore. Furthermore, the anthracene moiety should act as a hydrophobic surrounding for the amide binding site.

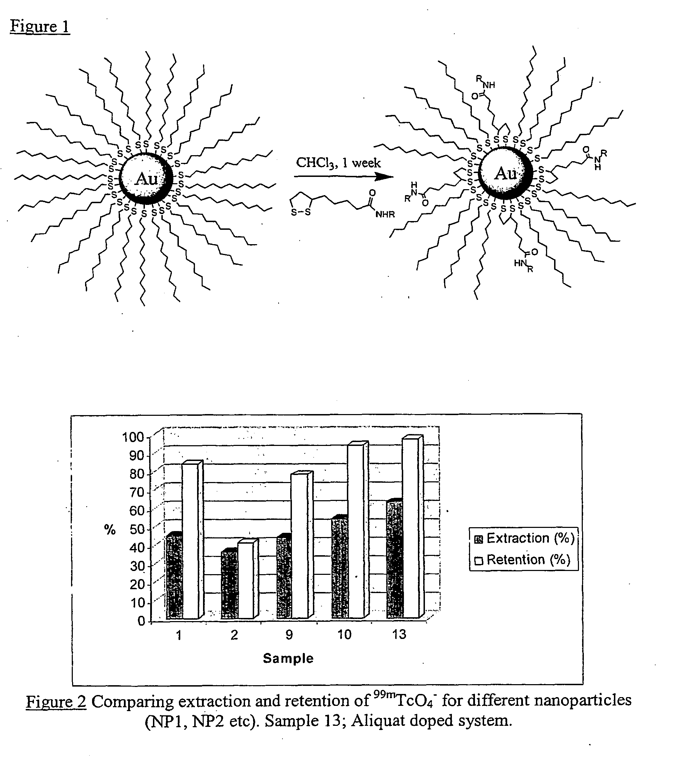

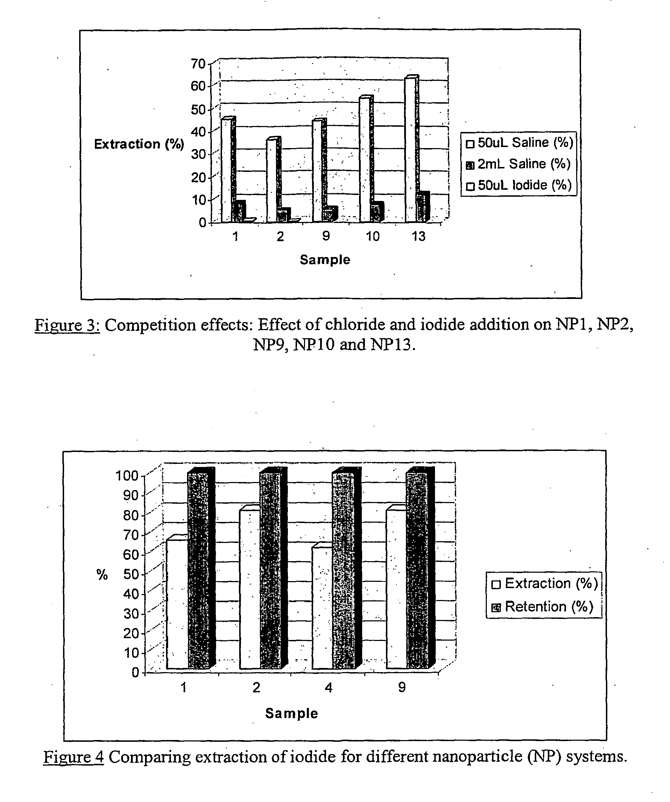

Example 3 provides the preparation of NPs having functionalised thiols attached. Example 4 provides a synthesis of thiols having an anion-binding site (a quaternary ammonium substituent). Example 5 shows how NPs having non-covalently bound ionic anion-binding sites can be obtained. Example 6 provides the nanoparticle radiolabelling procedure. Example 7 studies the stability of the radiolabelled NPs. This, together with Figures 2 to 4, show that (for pertechnetate):
- the presence of an amide ligand appears not to notably enhance pertechnetate extraction or retention;
- doping the nanoparticles with Aliquat substantially improves extraction and retention of pertechnetate anion;
- chloride acts as an effective competitor for pertechnetate, and, although it is in excess in all cases, the affinity for pertechnetate of the gold nanoparticles is significantly reduced in the presence of increased concentrations of aqueous chloride; the reduction is least marked in the case of NP13, wherein aliquat doping leads to a greater pertechnetate affinity. Furthermore, the presence of an excess of sodium iodide completely eliminates pertechnetate extraction, illustrating that the iodide anion competes more effectively with pertechnetate for the nanoparticle binding sites than does chloride.

Example 7 and Figures 2 to 4, show that (for radioiodide):
- Iodide extraction is enhanced by the presence of an amide ligand
- Retention of the chloroform-nanoparticle solutions is virtually quantitative, and independent of the ligand system - thus the extractions/associations are essentially irreversible.
- The presence of charged Aliquat doped nanoparticles substantially enhances extraction.

Example 8 shows that NP14 can be radiolabelled successfully with ¹²³I-iodide, but does not label ^{99m}Tc-pertechnetate. Example 9 shows that radiolabelled nanoparticles can be separated from free radioisotope by chromatography. The labelled nanoparticle can be separated from unbound label quite easily and within 10-15 minutes (including cartridge conditioning). Resultant fraction can then be diluted or concentrated to the desired radioactive concentration.

### Experimental.

The following abbreviations are used:
Boc = *tert*-butyloxycarbonyl.
DIPEA = Diisopropylethylamine.
DMF = N,N'-dimethylformamide.
HATU= *O*-(7-Azabenotriazol-1-yl)-*N,N,N,N'*-tetramethyluronium hexafluorophosphate.
ITLC = instant thin layer chromatography.
PEG = polyethylene glycol.
RAC = radioactive concentration.
RCP = radiochemical purity.
TFA = trifluoroacetic acid.

### General

All gold nanoparticle systems were characterised by using NMR and UV-visible spectroscopy, as well as elemental analysis. The presence of any ligand not grafted to the nanoparticle surface could be inferred from sharp peaks in the proton NMR spectrum. UV-visible spectra were demonstrated to contain surface plasmon bands, which are a characteristic feature of gold nanoparticle systems due to their small size [Kriebig et al, "Optical Properties of Metal Clusters "; Springer: Berlin, 1995]. The degree of substitution was calculated using simple C/H/N elemental analyses as the simple thiol-protected nanoparticles do not contain nitrogen, whereas the substituted systems do.

### Example 1: Preparation of Gold Nanoparticles with Thiol Coatings.

The Brust method [JCS, Chem. Commun., 801-802 (1994)] was utilised to form all the thiol-coated gold nanoparticle systems described. A wide range of organic thiols are commercially available. Gold-protected nanoparticles prepared in this invention were as follows:

**Table 1.**

| NP number | thiol |
|---|---|
| NP1 | dodecanethiol |
| NP2 | Compound 2 |
| NP4 | octadecanethiol |
| NP9 | Compound 5 |
| NP14 | Compound 11 |

| | |
|---|---|
| Note: Compounds 2 & 5 are described in Example 2. | |

For NP2, NP9 and NP14 it was found most convenient to first prepare NP1 then displace dodecanethiol with the new thiol, as is described in Example 3.

The UV absorbance maxima (in nm), corresponding to the surface plasmon resonance band of each nanoparticle, were: 497.6 (NP2), 501.0 (NP4), 498.4 (NP9) and 518.0 (NP 14).

### Example 2: Preparation of Amide-functionalised Thiols.

The amines used were largely commercially available - Step (b) provides syntheses for those which are not.

### Step (a): reaction of amines with thioctic acid.

The synthesis of a range of different disulfide-functionalised ligands containing amide hydrogen bonding groups was carried out in high yield (70-90%) via coupling racemic thioctic acid (Aldrich) with the appropriate amine in the presence of EDCI [1-(3-dimethylaminopropyl)-3-ethylcarbodiimide; Aldrich] - see Scheme 2 and Table 2. The thioctic amides (Compounds 2 to 10) were designed with a number of factors in mind, as discussed below, and substitution reactions with NP1 were investigated.

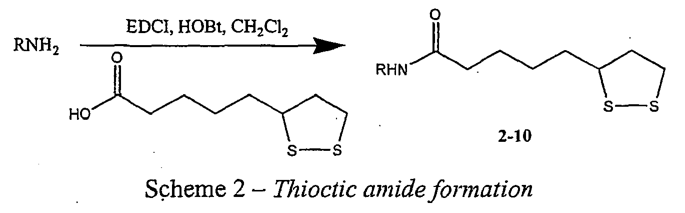

**Table 2 -Amides used for nanoparticle functionalisation**

| **Compound** | **Parent Amine (RNH₂)** |
|---|---|
| **2** | **R** = hexyl |
| **3** | **R** = tetradecyl |
| **4** | **R** = benzyl |
| **5** | **R** =*p-tert*-butylphenyl |
| **6** | 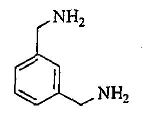 |
| **7** | 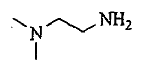 |
| **8** | 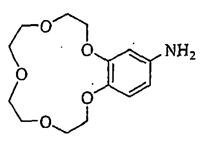 |
| **9** | 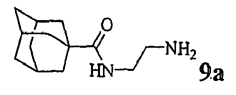 |
| **10** | 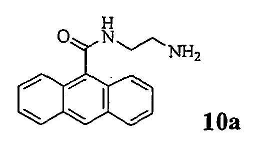 |
| **11** | 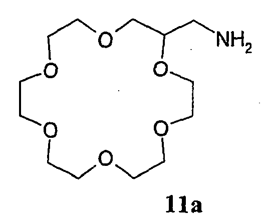 |

### Step (b): synthesis of amines.

The amine precursors for Compounds 9a and 10a were prepared *via* a partial-Boc protection strategy (Scheme 3):

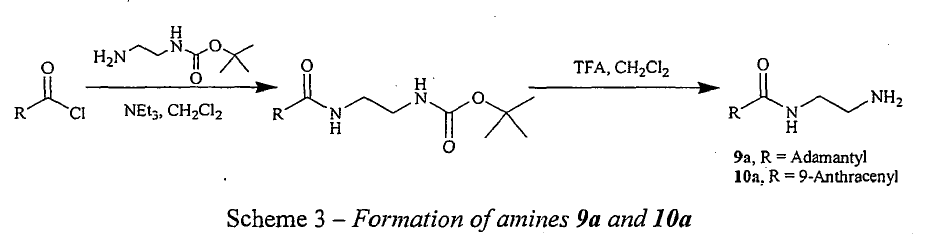

Mono-BOC protected ethylenediamine was prepared as previously reported [Fader et al J. Org. Chem., 66, 3372-3379 (2001)]. 1-adamantanecarbonyl chloride is commercially available. Anthracene-9-carbonyl chloride was prepared according to a literature procedure [Nakatsuji et al J. Org. Chem., 67, 916-921 (2002)]. Compound 11 a is commercially available (Sigma-Aldrich).

### Compound 2

¹H NMR (300MHz, CDCl₃) δ (ppm): 5.42 (br, 1H, N*H*), 3.51 (m, 1H, SC*H*), 3.01-3.24 (m, 4H, NHC*H₂* & SC*H₂*), 2.40 (m, 1H, SCH₂C*H₂*CH), 2.11 (t, ³J = 6.74, 2H, COC*H₂*), 1.85 (m, 1H, SCH₂C*H₂*CH), 1.62 (m, 4H, SCHC*H₂* & NHCH₂C*H₂*), 1.42 (bm, 4H, COCH₂C*H₂* & SCHCH₂C*H₂*), 1.22 (br, 6H, CH₃(C*H₂*)*₃*)*,* 0.82 (br, 6H, C*H₃*).

### Compound 3

¹H NMR (300MHz, CDCl₃) δ (ppm): 5.36 (br, 1H, N*H*), 3.50 (m, 1H, SC*H*), 3.01-3.20 (m, 4H, NHC*H₂* & SC*H₂*), 2.38 (m, 1H, SCH₂C*H*₂CH), 2.11 (t, ³J = 6.74, 2H, COC*H₂*), 1.84 (m, 1H, SCH₂C*H₂*CH), 1.61 (m, 4H, SCHC*H₂* & NHCH₂C*H₂*), 1.40 (m, 4H, COCH₂C*H₂* &. SCHCH₂C*H₂*), 1.22 (br, 22H, CH₃(C*H₂*)₁₁), 0.80 (br, 6H, C*H₃*).

### Compound 4

¹H NMR (500MHz, CDCl₃) δ (ppm): 7.36 (m, 2H, Ar*H*), 7.31 (m, 3H, Ar*H*), 5.80 (br, 1H, N*H*), 4.44 (d, ³J = 5.61 Hz, 2H, NHC*H*₂), 3.51 (dd, ³J₁ = 8.39Hz, ³J₂ = 6.25Hz, 1H, SC*H*), 3.10-3.19 (m, 2H, SC*H₂*), 2.44 (td, ²J = 12.46Hz, ³J = 6.62Hz,1H, SCH₂C*H₂*CH), 2.22 (t, ³J = 7.52, 2H, COC*H₂*), 1.89 (td, td, ²J = 12.87Hz, ³J = 6.83Hz, 1H, SCH₂C*H₂*CH), 1.68 (m, 4H, SCHC*H₂* & COCH₂C*H₂*), 1.47 (m, 2H, SCHCH₂C*H₂*).

### Compound 5

¹H NMR (500MHz, CDCl₃) δ (ppm): 7.45 (d, ³J = 8.54Hz, 2H, Ar*H*), 7.37 (d, ³J = 8.54Hz, 2H, Ar*H*), 7.24 (br, 1H, N*H*), 3.58 (in, 1H, SC*H*)*,* 3.06-3.23 (m, 2H, SC*H₂*), 2.45 (m,1H, SCH₂C*H₂*CH), 2.35 (t, ³J = 7.24, 2H, COC*H₂*),1.90 (td, ²J = 13.03Hz, ³J = 6.68Hz, 1H, SCH₂C*H₂*CH), 1.70 (m, 4H, SCHC*H₂* & COCH₂C*H₂*), 1.52 (m, 2H, SCHCH₂C*H₂*), 1.29 (s, 9H, C*H₃*).

### Compound 6

¹H NMR (300MHz, CDCl₃) δ (ppm): 7.34 (m, 2H, Ar*H*), 7.23 (m, 2H, Ar*H*), 5.86 (br, 2H, N*H*), 4.46 (d, ³J = 5.42Hz, NHC*H₂*), 3.60 (m, 2H, SC*H*), 3.18 (m, 4H, SC*H₂*), 2.48 (m, 2H, SCH₂C*H₂*CH), 2.27 (t, ³J = 7.26Hz, 4H, COC*H₂*), 1.95 (m, 2H, SCH₂C*H₂*CH), 1.73 (m, 8H, SCHC*H₂* & COCH₂C*H₂*), 1.51 (m, 4H, SCHCH₂C*H₂*).

### Compound 7

¹H NMR (300MHz, CDCl₃) δ (ppm): 6.03 (br, 1H, CON*H*), 3.65 (m, 1H, SC*H*), 3.26 (m, 2H, NHC*H₂*), 3.04-3.18 (m, 2H, SC*H₂*), 2.40 (m, 3H, SCH₂C*H₂*CH & (CH₃)₂NC*H₂*), 2.21 (s, 6H, NC*H₃*)*,* 2.17 (t, ³J = 7.93Hz, 2H, COC*H₂*), 1.84 (m, 2H, SCH₂C*H₂*CH), 1.61 (m, 4H, SCHC*H₂*, COCH₂C*H₂*), 1.42 (m, 2H, SCHCH₂C*H₂*).

### Compound 8

¹H NMR (300MHz, CDCl₃) δ (ppm): 7.40 (m, 1H, Ar*H*), 7.15 (br, 2H, N*H*), 6.85 (m, 2H, Ar*H*), 4.16 (m, 4H, ArOC*H₂*), 3.93 (m, 4H, ArOCH₂C*H₂*),3.80 (br, 8H, OC*H₂*), 3.62 (m, 1H, SC*H*), 3.11-3.26 (m, 2H, SC*H₂*), 2.49 (m, 1H, SCH₂C*H₂*CH), 2.38 (t, ³J = 7.63Hz, 2H, COC*H₂*), 1.96 (m, 1H, SCH₂C*H₂*CH), 1.78 (m, 4H, SCHC*H₂* & COCH₂C*H₂*), 1.55 (m, 2H, SCHCH₂C*H₂*.

### Compound 9

¹H NMR (300MHz, CDCl₃) δ (ppm): 6.61 (br, 1H, CON*H*), 6.48 (br, 1H, CON*H*), 3.62 (m, 1H, SC*H*), 3.40 (bm, 4H, 2 x NHC*H₂*), 3.09-3.24 (m, 2H, SC*H₂*), 2.47 (m, 1H, SCH₂C*H₂*CH), 2.29 (t, ³J = 7.61Hz, 2H, COC*H₂*), 2.03 (br, 3H, Adamantyl C*H*), 1.90 (m, 7H, SCH₂C*H₂*CH & COC(C*H₂*)₃), 1.74 (m, 10H, SCHC*H₂*, COCH₂C*H₂* & Adamantyl C*H₂*), 1.51 (m, 2H, SCHCH₂C*H₂*).

### Compound 10

¹H NMR (300MHz, CDCl₃) δ (ppm): 8.58 (s, 1H, (ArC)₂C*H*), 8.02 (m, 4H, ArCC*H*CH), 8.57 (m, 4H, ArCCHC*H*), 6.78 (br, 1H, CONH), 6.60 (br, 1H, CONH), 3.95 (m, 2H, C*H₂*NH), 3.63 (m, 2H, C*H₂*NH), 3.49 (m, 1H, SC*H*), 3.04-3.22 (m, 2H, SC*H₂*), 2.42 (m,1H, SCH₂C*H₂*CH)*,* 2.23 (t, ³J = 7.10, 2H, COC*H*₂), 1.87 (m, 1H, SCH₂C*H₂*CH), 1.65 (m, 4H, SCHC*H₂* & COCH₂C*H₂*), 1.47 (m, 2H, SCHCH₂C*H₂*).

### Compound 11

¹H NMR (300MHz, CDCl₃) δ (ppm): 6.27 (br, 1H, N*H*), 3.40 - 3.80 (m, 24H, OC*H₂*, OC*H*, SC*H*)*,* 3.27 (m, 2H, NHC*H*₂), 3.00-3.16 (m, 2H, SC*H₂*), 2.49 (m, 1H, SCH₂C*H₂*CH), 2.13 (t, ³J = 7.33Hz, 2H, COC*H₂*), 1.85 (m, 1H, SCH₂C*H₂*CH), 1.61 (m, 4H, SCHC*H₂* & COCH₂C*H₂*), 1.40 (m, 2H, SCHCH₂C*H₂*).

### Compound 9a

¹H NMR (300MHz, CDCl₃) δ (ppm): 6.24 (br, 1H, CON*H*), 3.35 (bm, 2H, NHC*H₂*), 2.89 (t, ³J = 5.27Hz), 2.07 (br, 5H, CH₂N*H₂* & Adamantyl C*H*), 1.89 (COC(C*H₂*)₃), 1.75 (Adamantyl C*H₂*).

### Compound 10a

¹H NMR (300MHz, CDCl₃) δ (ppm): 8.36 (s, 1H, (ArC)₂C*H*), 7.91 (m, 4H, ArCC*H*CH), 7.40 (m, 4H, ArCCHC*H*), 6.56 (br, 1H, CON*H*), 3.58 (q, ³J = 5.86Hz, 2H, CONHC*H*₂), 2.91 (t, ³J = 5.94Hz, 2H, NH₂C*H₂*), 1.24 (br, 2H, CH₂N*H₂*).

### Example 3: Preparation of Nanoparticles having Functionalised Thiols.

Nanoparticles based on functionalised thiols were prepared by substitution reactions of NP1 with appropriate thioctic acid derived disulfide ligands using the method of Beer et al [JCS J. Chem. Commun., 414-415 (2004)]. This entails stirring the nanoparticle and functionalised thiol for a week under chloroform, then washing the resulting precipitate exhaustively with acetone (or any other appropriate solvent) to remove excess unreacted ligand (see Figure 1). Where substitution was not initially observed to occur, excess NaBH₄ was added to encourage reaction by reduction of the disulfide linkage.

The results of the attempted substitution reactions with dodecanethiol-protected nanoparticle NP1, for the amides of Example 2 are summarised in Table 3 below. Most systems, as indicated, proved amenable to the substitution methodology. In the case of the crown system Compound 8, reaction was only observed to occur in the presence of a borohydride reducing agent. The resulting nanoparticle product, however, was methanol soluble which indicates the possibility of crown ether coordination to the sodium cation. The nanoparticles produced by substitution of Compound 7, on the other hand, proved to be highly insoluble in all potential solvent systems investigated, and thus could not be properly purified.

**Table 3**

| **Compound** | **Reaction with NP1?** |
|---|---|
| **2** | Yes |
| **3** | Yes |
| **4** | Yes |
| **5** | Yes |
| **6** | Yes |
| **7** | Product insoluble |
| **8** | Yes Product soluble in methanol |
| **9** | Yes |
| **11** | Yes |

### Example 4: Thiols Functionalised with Quaternary Ammonium Salts.

A literature procedure was adapted to produce the trimethylammonium thiol **41** via the disulphide **40** (Scheme 4) [Ekambarm et al, J.Org.Chem., 32,-2985-2987 (1967)]:

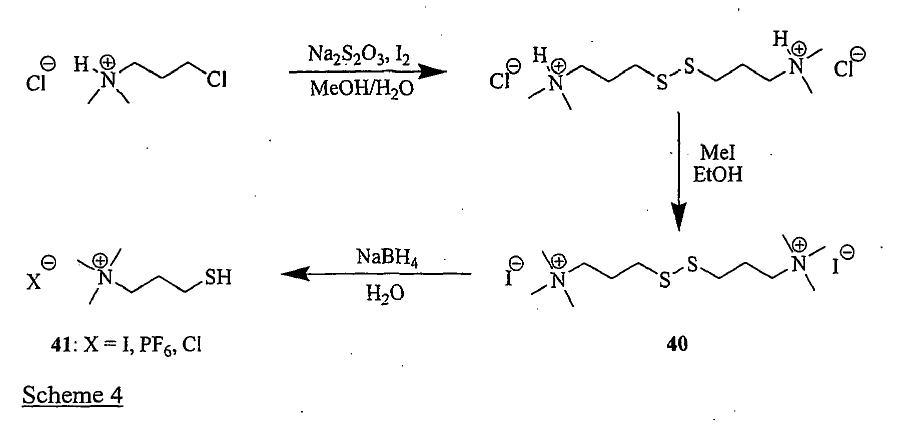

### Example 5: Nanoparticles Doped with Quaternary Ammonium Salts.

The nanoparticles NP1 doped with Aliquat^{®} 336 chloride (Aldrich) were prepared by mixing different mass ratios of NP1 nanoparticles and Aliquat^{®} 336 chloride in chloroform solution. The association of the Aliquat and nanoparticles was reversible, as exhaustive washing of the resulting systems with methanol and acetone was able to remove the Aliquat cations from the nanoparticles, as indicated by ¹H NMR spectroscopy. A slight broadening of the Aliquat^{®} proton NMR resonances suggested that they were indeed associated with the nanoparticle surface.

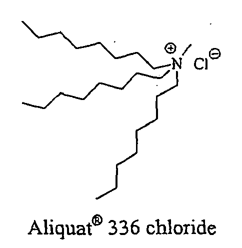

The nanoparticles having a molar ratio of approximately one Aliquat^{®} 336 chloride molecule per NP were dubbed NP13 (this corresponds to a molar ratio of approximately 1:10 Aliquat: organic thiol).

### Example 6: Nanoparticle Radiolabelling Procedure.

100µL of a 1mg/cm³ CHCl₃ solution of nanoparticle NP1 was diluted to 2 cm³ with chloroform, to give a nanoparticle concentration of 50µg per mL^{‡}. To this solution was then added 1.95 cm³ of either aqueous (AnalaR water) or salt solution (0.9% or 0.023% w/v), depending on the experiment*. To the resultant mixture a 50uL solution of the appropriate radioactive anion^{†} was added. The resultant mixture was 'whirlimixed' for 20 seconds, then centrifuged for 30 minutes, in order to aid phase separation. The phases were then partitioned by pipette, and the radionuclide content of 1 cm³ samples of both the aqueous and chloroform layers were assayed using a Wallac emission detector. From the resulting counts, the percentage distribution of the relevant radioisotope could be obtained, and by inference, the total ion distribution. All experiments were conducted in duplicate.
^{‡}Concentration was varied in some experiments.
*For 0.023% w/v solutions, 50uL of a 0.9% salt (either NaCl or NaI) solution was diluted to the appropriate 1.95mL by water.
^{†} Pertechnetate used = ^{99m}TcO₄⁻ . Iodide =¹²³I⁻. Na⁺ was the counterion in both cases. The molarity was calculated from radioactivity information. Activity was added as 50µL of a 4MBq/mL solution of radioisotope. Solvents: 0.9% w/v NaCl for ^{99m}Tc, 1M NaOH for ¹²³I.

### Example 7: Stability of Radiolabelled Nanoparticles.

This was established via a back extraction procedure: To a 500µL solution of the organic layer obtained from Example 6 was added an equal volume of water or saline solution (0.9% w/v). The resulting mixture was 'whirlimixed' for 20 seconds, then centrifuged for 30 minutes to aid phase separation. The radionuclide content of 100µL portions of the resulting phases were then assayed in an analogous manner to that detailed above. It is worth noting that, as a consequence of isotope decay and sample size, the total counts recorded in these experiments were much lower, and thus the inherent error larger. All experiments were conducted in duplicate.
The results are shown in Figures 2 to 4.

### Example 8: Radiolabelling of Nanoparticle 14 (NP14).

NP14 was dissolved in water to 0,5 mg/mL dilution. Further dilution, as required, was accomplished by adding water to these solutions. For all radiolabelling experiments, 200µL of aqueous nanoparticle solution was treated with 20µL of a solution of the active radioisotope in an Eppendorf cuvette. The activity was approximately 30MBq per 20µL of isotope solution added. The resulting solutions were mixed by pipette, and the activity of the sample measured in an ion chamber. This allowed the calculation of the amount of radioisotope present. Typical preparations follow.

### (a) ^{99m}Tc-Pertechnetate.

50µL of a generator-derived solution of Na^{99m}TcO₄ in 0.9 w/v saline solution (activity: 131 MBq) was diluted with 5µL of H₂O. 4 x 20µL portions of this solution were then added to the 200µL portions of the aqueous NP14 solution. The samples were mixed by pipette, and the activity measured after 10 minutes. This was 27.6MBq. The samples were allowed to equilibrate for a further ten minutes, then analysed by ITLC and HPLC (see later sections). The overall 220µL solutions contained 0.041% w/v saline.

### (b) ¹²³I-Iodide.

7µL of a 0.05M NaOH_{(aq)} solution of Na¹²³I (activity: 150MBq) was diluted to 100µL with 0.01M NaOH_{(aq)}. 5 x 20µL portions of this solution were then added to the 200µL portions of the aqueous nanoparticle solutions, and one 'bank' 200µL water solution as a control. The samples were mixed by pipette and the activity measured after 5 minutes. This was 27.2MBq, Blank, 23.6MBq. The samples were allowed to equilibrate for a further ten minutes, then analysed by ITLC (see below). The overall 220µL solutions were 0.233mM NaOH_{(aq)}.

### (c) Analytical Procedures.

### (i) ITLC.

*ca* 5µL of the radiolabelled sample was spotted onto an ITLC strip (silica gel impregnated glass fibre sheets). The strip was approx 20cm in length. This was then eluted with 0.9 w/v saline solution, until the eluant had nearly reached the top of the . TLC strip. The samples were allowed to dry, then placed onto imaging plates and scanned in a Perkin-Elmer InstantImager scanner. The gold nanoparticles remain on the baseline (rf = 0) under these conditions, whereas free NaTcO₄ and NaI elute with the solvent (rf∼1).

### (ii) Sephadex G-25.

A NAP-5 column was equilibrated using 10mL of a 10mM sodium phosphate buffer solution in 0.9% w/v saline solution. To this was added 200µL of the radiolabelled solution, along with 300µL of the eluant (10mM sodium phosphate buffer solution in 0.9% w/v saline solution). This was allowed to enter the Sephadex, and then the column was eluted with 1mL of buffer solution. The first 1mL eluted was collected, with no further fractions obtained. The activity of the eluted volume could then be measured using an ion chamber, and compared to the original activity of the crude sample. In a control experiment, Na¹²³I did not appreciably elute from the column.

### (d) Results.

No uptake of pertechnetate was observed by NP14. Uptake was, however, observed for ¹²³I-iodide:

**Table 4: Uptake of iodide by NP14 as a function of time and concentration.**

| | Concentration:0.5mg/mL | | Concentration:5mg/mL | |
|---|---|---|---|---|
| Time (min)¹ | % labelled | % free | % labelled | % free |
| 15 | 2.4 | 93.3 | 5.1 | 91.1 |
| 60 | 4.6 | 90.7 | 10.4 | 84.9 |
| 150 | 6 | 88.9 | 12.5 | 81.4 |
| 360 | 7 | 87.8 | 14.3 | 79.1 |

| | | | | |
|---|---|---|---|---|
| Time taken from mixing of nanoparticles with radiolabel. | | | | |

### Example 9: Purification of ¹²³I-labelled Nanoparticle 14 (NP14).

An aqueous sample of ¹²³I-labelled NP14 (Example 8) which had been allowed to equilibrate with the iodide radiolabel for 1 hour, was subjected to Sephadex G-25 chromatography as per Example 8 giving a yield of 5.1%.

## Claims

1. A radiolabelled nanoparticle which comprises a nanoparticle having:
(i) a metallic core which comprises copper, silver, palladium or gold or combinations thereof;
(ii) a lipophilic coating around said core which comprises a multiplicity of C₂₋₂₅ organic thiols bound to said core, wherein said thiols may be the same or different and may be in reduced (ie. thiol) or oxidised (ie. disulfide) form or combinations thereof;
which is labelled with at least one radioisotope which is non-covalently bonded to said nanoparticle.

2. The nanoparticle of Claim 1, where the organic thiol is in the reduced (ie. thiol) form.

3. The nanoparticle of Claims 1 or 2, where the metallic core comprises gold.

4. The nanoparticle of any one of Claims 1 to 3, which further comprises a biological targeting moiety.

5. The nanoparticle of Claim 4, where the biological targeting moiety comprises a peptide, protein, enzyme substrate, enzyme antagonist or enzyme inhibitor.

6. The nanoparticle of Claims 4 or 5, where the biological targeting moiety comprises a thiol functional group which is bound to the metallic core.

7. The nanoparticle of any one of Claims 1 to 6, where the lipophilic coating comprises a proportion of thiols which further comprise one or more anion-binding substituents.

8. The nanoparticle of Claim 7, where the anion-binding substituent is positively charged, and is of Formula -ER1¹₃⁺ X⁻, where:
E is N or P;
R¹ is C₁₋₁₀ alkyl, which may be linear or branched; C₂₋₁₀ alkoxyalkyl; C₂₋₁₂ aryl or C₂₋₁₂ heteroaryl;
X is Hal, OH, PF₆, H₂PO₄, nitrate, C₁₋₈ carboxylate or C₁₋₈ sulfonate.

9. The nanoparticle of any one of Claims 1 to 6, where the lipophilic coating comprises a proportion of thiols which further comprise one or more cation-binding substituents.

10. The nanoparticle of any one of Claims 1 to 9, where the thiol is of Formula R²SH or R²S-SR², wherein R² is C₅₋₂₄ alkyl, C₅₋₂₄ aralkyl, or C₅₋₁₂ aryl, and R² may optionally be substituted with one or more anion-binding or cation-binding substituents.

11. The nanoparticle of any one of Claims 1 to 10, which further comprises an organic cation chosen from quaternary ammonium salts, phosphonium salts, imidazolium, uronium, or other biocompatible organic cation molar ratio of about 1:5 to 1:20 [organic cation] : [organic thiol].

12. The nanoparticle of any one of Claims 1 to 11, where the radioisotope is suitable for radiopharmaceutical imaging of the mammalian body *in vivo.*

13. The nanoparticle of any one of Claims 1 to 11, where the radioisotope is suitable for radiopharmaceutical therapy of the mammalian body *in vivo.*

14. The nanoparticle of Claims 12 or 13, where the radioisotope comprises ^{99m}Tc, ^{94m}Tc ¹⁸⁶Re, ¹⁸⁸Re, ¹²³I, ¹²⁴I, ¹²⁵I or ¹³¹I.

15. The nanoparticle of Claim 14, where the chemical form of the radioisotope is:
(i) pertechnetate for technetium radioisotopes;
(ii) perrhenate for rhenium radioisotopes;
(iii) iodide ion for iodine radioisotopes.

16. A radiopharmaceutical composition which comprises a plurality of the radiolabelled nanoparticles of Claims 1 to 15 together with a biocompatible carrier, in a form suitable for mammalian administration.

17. The radiopharmaceutical composition of Claim 16, which has a radioactive dose suitable for a single patient and is provided in a suitable syringe or container.

18. A method of preparation of the radiolabelled nanoparticle of Claims 1 to 15, which comprises:
(i) provision of non-radioactive, unlabelled nanoparticles as defined in Claims 1 to 11;
(ii) optional purification of the nanoparticles from step (i);
(iii) reaction of the pre-formed nanoparticles from step (i) or step (ii) with a source of the radioisotope, such that the radioisotope is non-covalently bound to the nanoparticle.

19. A kit for the preparation of the radiopharmaceutical composition of Claims 16 or 17, which comprises non-radioactive, unlabelled nanoparticles as defined in Claims 1 to 10.

20. The kit of Claim 19, where the unlabelled nanoparticles are in sterile, apyrogenic form.

21. Use of the radiolabelled nanoparticles of Claims 1 to 15 in the manufacture of a medicament for use in radiopharmaceutical imaging *in vivo.*

22. Use of the radiolabelled nanoparticles of Claims 1 to 15 in the manufacture of a medicament for use in radiopharmaceutical therapy *in vivo*.

## Patentansprüche

1. Radiomarkiertes Nanoteilchen, das ein Nanoteilchen umfasst mit:
(i) einem metallischen Kern, welcher Kupfer, Silber, Palladium oder Gold oder Kombinationen daraus umfasst;
(ii) einer lipophilen Beschichtung um den Kern, die eine Vielzahl organischer C₂₋₂₅-Thiole umfasst, die an den Kern gebunden sind, wobei die Thiole gleich oder verschieden sein können und in reduzierter (d.h. Thiol-) oder oxidierter (d.h. Disulfid-) Form oder Kombinationen daraus vorliegen können;
das mit zumindest einem Radioisotop markiert ist, das nicht-kovalent an das Nanoteilchen gebunden ist.

2. Nanoteilchen nach Anspruch 1, wobei das organische Thiol in der reduzierten (d.h. Thiol-) Form vorliegt.

3. Nanoteilchen nach Anspruch 1 oder 2, wobei der metallische Kern Gold umfasst.

4. Nanoteilchen nach einem der Ansprüche 1 bis 3, das weiterhin einen auf ein biologisches Ziel gerichteten Teil umfasst.

5. Nanoteilchen nach Anspruch 4, wobei der auf ein biologisches Ziel gerichtete Teil ein Peptid, ein Protein, ein Enzymsubstrat, einen Enzymantagonisten oder einen Enzyminhibitor umfasst.

6. Nanoteilchen nach Anspruch 4 oder 5, wobei der auf ein biologisches Ziel gerichtete Teil eine funktionelle Thiolgruppe umfasst, die an den metallischen Kern gebunden ist.

7. Nanoteilchen nach einem der Ansprüche 1 bis 6, wobei die lipophile Beschichtung einen Anteil von Thiolen umfasst, die weiterhin einen oder mehrere anionenbindende Substituenten umfassen.

8. Nanoteilchen nach Anspruch 7, wobei der anionenbindende Substituent positiv geladen ist, und die Formel -ER¹₃⁺X⁻ hat, wobei:
E für N oder P steht;
R¹ für C₁₋₁₀-Alkyl, das linear oder verzweigt sein kann; C₂₋₁₀-Alkoxyalkyl; C₂₋₁₂-Aryl oder C₂₋₁₂-Heteroaryl steht;
X für Hal, OH, PF₆, H₂PO₄, Nitrat, C₁₋₈-Carboxylat oder C₁₋₈-Sulfonat steht.

9. Nanoteilchen nach einem der Ansprüche 1 bis 6, wobei die lipophile Beschichtung einen Anteil von Thiolen umfasst, die weiterhin einen oder mehrere kationenbindende Substituenten umfassen.

10. Nanoteilchen nach einem der Ansprüche 1 bis 9, wobei das Thiol die Formel R²SH oder R²S-SR² hat, wobei R² für C₅₋₂₄-Alkyl, C₅₋₂₄-Aralkyl oder C₅₋₁₂-Aryl steht, und R² gegebenenfalls substituiert sein kann durch einen oder mehrere anionenbindende oder kationenbindende Substituenten.

11. Nanoteilchen nach einem der Ansprüche 1 bis 10, das weiterhin ein organisches Kation, ausgewählt aus quartären Ammoniumsalzen, Phosphoniumsalzen, Imidazolium, Uronium, oder ein anderes biokompatibles organisches Kation umfasst bei einem Molverhältnis von ungefähr 1:5 bis 1:20 [organisches Kation] : [organisches Thiol].

12. Nanoteilchen nach einem der Ansprüche 1 bis 11, wobei das Radioisotop zur radiopharmazeutischen Bildgebung des Säugetierkörpers *in vivo* geeignet ist.

13. Nanoteilchen nach einem der Ansprüche 1 bis 11, wobei das Radioisotop zur radiopharmazeutischen Therapie des Säugetierkörpers *in vivo* geeignet ist.

14. Nanoteilchen nach Anspruch 12 oder 13, wobei das Radioisotop ^{99m}Tc, ^{94m}Tc, ¹⁸⁶Re, ¹⁸⁸Re, ¹²³I, ¹²⁴I, ¹²⁵I oder ¹³¹I umfasst.

15. Nanoteilchen nach Anspruch 14, wobei die chemische Form des Radioisotops lautet:
(i) Pertechnetat für Technetiumradioisotope;
(ii) Perrhenat für Rheniumradioisotope;
(iii) Iodidion für Iodradioisotope.

16. Radiopharmazeutische Zusammensetzung, die eine Mehrzahl der radiomarkierten Nanoteilchen nach Anspruch 1 bis 15 zusammen mit einem biokompatiblen Träger umfasst, und zwar in einer zur Verabreichung an Säugetiere geeigneten Form.

17. Radiopharmazeutische Zusammensetzung nach Anspruch 16, die eine radioaktive Dosis aufweist, die für einen einzelnen Patienten geeignet ist und in einer zweckmäßigen Spritze oder einem zweckmäßigen Behälter zur Verfügung gestellt wird.

18. Verfahren zur Herstellung des radiomarkierten Nanoteilchens nach Anspruch 1 bis 15, welches umfasst:
(i) Bereitstellung nicht radioaktiver, unmarkierter Nanoteilchen, wie in Anspruch 1 bis 11 definiert;
(ii) eventuelle Reinigung der Nanoteilchen aus Schritt (i);
(iii) Umsetzung der vorgeformten Nanoteilchen aus Schritt (i) oder Schritt (ii) mit einer Quelle des Radioisotops, so dass das Radioisotop nicht-kovalent an das Nanoteilchen gebunden ist.

19. Kit zur Herstellung der radiopharmazeutischen Zusammensetzung nach Anspruch 16 oder 17, welches nicht radioaktive, unmarkierte Nanoteilchen umfasst, wie in Anspruch 1 bis 10 definiert.

20. Kit nach Anspruch 19, wobei die unmarkierten Nanoteilchen in steriler, apyrogener Form sind.

21. Verwendung der radiomarkierten Nanoteilchen nach Anspruch 1 bis 15 bei der Herstellung eines Medikaments zum Einsatz bei radiopharmazeutischer Bildgebung *in vivo.*

22. Verwendung der radiomarkierten Nanoteilchen nach Anspruch 1 bis 15 bei der Herstellung eines Medikaments zum Einsatz bei radiopharmazeutischer Therapie *in vivo.*

## Revendications

1. Nanoparticule marquée par radio-isotope qui comprend une nanoparticule comportant :
(i) un noyau métallique qui comporte du cuivre, de l'argent, du palladium ou de l'or ou un mélange de ceux-ci ;
(ii) un revêtement lipophile autour dudit noyau qui comprend une multiplicité de thiols organiques en C₂₋₂₅ liés audit noyau, dans lequel lesdits thiols peuvent être identiques ou différents et peuvent être sous forme réduite (c'est-à-dire, de thiol) ou oxydée (c'est-à-dire, de bisulfure) ou d'une association de ces dernières ;
qui est marquée avec au moins un radio-isotope qui est lié de manière non covalente à ladite nanoparticule.

2. Nanoparticule selon la revendication 1, dans laquelle le thiol organique est sous la forme réduite (c'est-à-dire, de thiol).

3. Nanoparticule selon les revendications 1 ou 2, dans laquelle le noyau métallique comporte de l'or.

4. Nanoparticule l'une quelconque des revendications 1 à 3, comportant, en outre, une fraction de ciblage biologique.

5. Nanoparticule selon la revendication 4, dans laquelle la fraction de ciblage biologique comprend un peptide, une protéine, un substrat d'enzyme, un antagoniste d'enzyme ou un inhibiteur d'enzyme.

6. Nanoparticule selon les revendications 4 ou 5, dans laquelle la fraction de ciblage biologique comprend un groupe fonctionnel thiol qui est lié au noyau métallique.

7. Nanoparticule selon l'une quelconque des revendications 1 à 6, dans laquelle le revêtement lipophile comprend une proportion de thiols qui comprennent, en outre, un ou plusieurs substituants de liaison d'anion.

8. Nanoparticule selon la revendication 7, dans laquelle le substituant de liaison d'anion est chargé positivement, et présente la formule -ER¹₃⁺X⁻, dans laquelle :
E représente N ou P ;
R¹ représente un alkyle en C₁₋₁₀, qui peut être linéaire ou ramifié ; un alkoxyalkyle en C₂₋₁₀ ; un aryle en C₂₋₁₂ ou hétéroaryle en C₂₋₁₂ ;
X représente Hal, OH, PF₆, H₂PO₄, un nitrate, un carboxylate en C₁₋₈ ou un sulfonate en C₁₋₈.

9. Nanoparticule selon l'une quelconque des revendications 1 à 6, dans laquelle le revêtement lipophile comprend une proportion de thiols qui comprennent, en outre, un ou plusieurs substituants de liaison de cation.

10. Nanoparticule selon l'une quelconque des revendications 1 à 9, dans laquelle le thiol présente la formule R²SH ou R²S-SR², dans laquelle R² est un alkyle en C₅₋₂₄, un arylalkyle en C₅₋₂₄, ou un aryle en C₅₋₁₂, et R² peut en variante être substitué avec un ou plusieurs substituants de liaison d'anion ou de liaison de cation.

11. Nanoparticule selon l'une quelconque des revendications 1 à 10, comprenant, en outre, un cation organique choisi à partir de sels d'ammonium quaternaire, de sels de phosphonium, d'imidazolium, d'uronium, ou autre cation organique biocompatible avec un rapport molaire de 1:5 à 1:20 environ [cation organique] : [thiol organique].

12. Nanoparticule selon l'une quelconque des revendications 1 à 11, dans laquelle le radio-isotope est approprié à l'imagerie radio-pharmaceutique du corps de mammifère in vivo.

13. Nanoparticule selon l'une quelconque des revendications 1 à 11, dans laquelle le radio-isotope est approprié à la thérapie radio-pharmaceutique du corps de mammifère in vivo.

14. Nanoparticule selon les revendications 12 ou 13, dans laquelle le radio-isotope comprend les éléments ^{99m}Tc, ^{94m}Tc ¹⁸⁶Re, ¹⁸⁸Re, ¹²³I, ¹²⁴I, ¹²⁵I ou ¹³¹I.

15. Nanoparticule selon la revendication 14, dans laquelle la forme chimique du radio-isotope est :
(i) un pertechnétate pour des radio-isotopes à base de technétium ;
(ii) un perrhénate pour des radio-isotopes à base de rhénium ;
(iii) un ion iodure pour des radio-isotopes à base d'iode.

16. Composition radio-pharmaceutique qui comprend une pluralité des nanoparticules marquées par radio-isotope selon les revendications 1 à 15, ensemble avec un support biocompatible, sous une forme appropriée pour l'administration à un mammifère.

17. Composition radio-pharmaceutique selon la revendication 16, qui comporte une dose radioactive appropriée pour un seul patient et est délivrée dans une seringue ou conteneur approprié.

18. Procédé de préparation de nanoparticule marquée par radio-isotope selon les revendications 1 à 15, qui comprend :
(i) préparation de nanoparticules non marquées, non radioactives selon l'une des revendications 1 à 11 ;
(ii) purification éventuelle des nanoparticules provenant de l'étape (i) ;
(iii) réaction des nanoparticules formées préalablement à partir de l'étape (i) ou de l'étape (ii) avec une source du radio-isotope, de telle sorte que le radio-isotope se lie de manière non covalente à la nanoparticule.

19. Kit de préparation de la composition radio-pharmaceutique selon les revendications 16 ou 17, qui comprend des nanoparticules non marquées, non radioactives selon les revendications 1 à 10.

20. Kit selon la revendication 19, dans lequel les nanoparticules non marquées sont sous une forme stérile et apyrogène.

21. Utilisation des nanoparticules marquées par radio-isotope selon les revendications 1 à 15, dans la fabrication d'un médicament en vue d'une utilisation en imagerie radio-pharmaceutique in vivo.

22. Utilisation des nanoparticules marquées par radio-isotope selon les revendications 1 à 15, dans la fabrication d'un médicament en vue d'une utilisation en thérapie radio-pharmaceutique in vivo.
